(19) **Europäisches Patentamt European Patent Office Office européen des brevets**

(11) **EP 3 197 850 B1**

(12) **FASCICULE DE BREVET EUROPEEN**

(45) Date de publication et mention de la délivrance du brevet:
**21.11.2018 Bulletin 2018/47**

(21) Numéro de dépôt: **15770500.5**

(22) Date de dépôt: **22.09.2015**

(51) Int Cl.:
*C07C 1/24* (2006.01)     *C07C 5/27* (2006.01)
*B01J 29/65* (2006.01)     *C07C 11/08* (2006.01)
*C07C 11/09* (2006.01)     *B01J 35/10* (2006.01)

(86) Numéro de dépôt international:
**PCT/EP2015/071802**

(87) Numéro de publication internationale:
**WO 2016/046242 (31.03.2016 Gazette 2016/13)**

(54) **PROCEDE DE DESHYDRATATION ISOMERISANTE D'UNE CHARGE ALCOOL PRIMAIRE SUBSTITUE EN POSITION 2 PAR UN GROUPEMENT ALKYL SUR UN CATALYSEUR COMPRENANT UNE ZEOLITHE DE TYPE FER**

VERFAHREN ZUR ISOMERISIERUNG VON DEHYDRATISIERUNG EINES PRIMÄREN ALKOHOLS IN 2-POSITION SUBSTITUIERT DURCH EINE ALKYLGRUPPE AN EINEM KATALYSATOR, UMFASSEND EIN EISENZEOLITH

METHOD FOR ISOMERIZING DEHYDRATION OF A PRIMARY ALCOHOL SUBSTITUTED IN POSITION 2 BY AN ALKYL GROUP ON A CATALYST COMPRISING AN IRON ZEOLITE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **26.09.2014 FR 1459113**

(43) Date de publication de la demande:
**02.08.2017 Bulletin 2017/31**

(73) Titulaires:
- **IFP Energies nouvelles
  92500 Rueil-Malmaison (FR)**
- **Total Research & Technology Feluy
  7181 Seneffe (BE)**

(72) Inventeurs:
- **VIVIEN, Tom
  F-69007 Lyon (FR)**
- **MAURY, Sylvie
  F-69440 Saint Maurice D'argoire (FR)**
- **COUPARD, Vincent
  F-69100 Villeurbanne (FR)**

- **BAZER-BACHI, Delphine
  F-30340 Saint Privat des Vieux (FR)**
- **NESTERENKO, Nikolai
  B-14000 Nivelles (BE)**
- **DANILINA, Nadiya
  B-1180 Uccle (BE)**

(74) Mandataire: **IFP Energies nouvelles
Rond-point de l'échangeur de Solaize
BP3
69230 Solaize (FR)**

(56) Documents cités:
**WO-A1-2011/113834     WO-A1-2013/011208**

- **CANIZARES P ET AL: "Isomerization of n-butene over ferrierite zeolite modified by silicon tetrachloride treatment", APPLIED CATALYSIS A: GENERAL, ELSEVIER SCIENCE, AMSTERDAM, NL, vol. 190, no. 1-2, 3 janvier 2000 (2000-01-03), pages 93-105, XP004272075, ISSN: 0926-860X, DOI: 10.1016/S0926-860X(99)00265-3**

EP 3 197 850 B1

**Description**

**DOMAINE TECHNIQUE DE L'INVENTION**

La présente invention concerne un procédé amélioré de production d'alcènes à partir d'une charge comprenant un monoalcool primaire substitué en position 2 par un groupement alkyl. Cette charge peut être obtenue par des procédés chimiques ou par des procédés fermentaires. Ce procédé met en oeuvre un catalyseur mis en forme à base d'une zéolithe de type structural FER et pré-coké.

Les alcènes obtenus, en particulier l'isobutène, le butène-1 et les butènes-2, présentent un intérêt important dans le domaine de l'industrie pétrochimique et de la synthèse organique.

**ART ANTÉRIEUR**

L'isobutène est une molécule clé en pétrochimie et pour la synthèse d'additifs essence tels que l'ETBE et le MTBE. La grande majorité des publications portent sur la production d'isobutène à partir de butanols linéaires, Ceux-ci étant plus facilement produits par voies fermentaires classiques (ABE) que l'isobutanol. De récents développements ont cependant permis d'améliorer fortement les rendements fermentaires en isobutanol, rendant cette charge accessible et disponible à coût attractif.

Le document WO2009/079213 décrit l'enchainement de réactions de déshydratation d'alcools (C2-C7) biosourcés sur un catalyseur acide pour former des oléfines suivies de l'oligomérisation des oléfines sur un catalyseur d'oligomérisation acide (zéolithe ou alumine). L'application visée est la préparation de carburant Jet fuel.

Le document EP 2348 005 décrit la déshydratation d'alcools contenant de 2 à 10 atomes de carbone en l'oléfine correspondante sur un catalyseur zéolithique FER de ratio atomique Si/Al inférieur à 100. La vitesse spaciale horaire massique (Weight Hourly Space Velocity selon la dénomination anglaise, ou WHSV) par rapport à l'alcool est d'au moins 4 $h^{-1}$ et la température de 320 à 600°C.

Le document WO 2011/089235 étend cette invention à d'autres types structuraux de zéolithes appartenant tous à la famille des zéolithes à taille de canaux moyenne (10MR), de ratio molaire Si/Al inférieur à 100. Les zéolithes peuvent être modifiées par différents post traitements. L'inventeur revendique la déshydratation d'alcools contenant de 2 à 10 atomes de carbone en l'oléfine correspondante.

Le document WO 2011/113834 décrit la déshydratation et l'isomérisation squelettale simultanée de l'isobutanol en présence de catalyseurs silicates cristallins, à taille de canaux moyenne (10MR) déaluminisés ou non, modifiés au phosphore ou non, du groupe FER, MWW, EUO, MFS, ZSM-48, MTT, MFI, MEL ou TON ayant un ratio Si/Al supérieur à 10, tamis moléculaires silicoaluminophosphates du groupe AEL, ou silice-, zircone-, titane- ou fluor-alumine. sur catalyseurs zéolithiques. La WHSV par rapport à l'alcool est d'au moins 1 $h^{-1}$ et la température de 200 à 600°C. La proportion maximale atteinte en n-butènes dans les butènes est de 58.4% à 375°C à forte WHSV (12.6 $h^{-1}$) sur une zéolithe FER en poudre de Si/Al 33. Aucune notion de stabilité de ces performances en fonction du temps sous charge n'est évoquée dans ce document. Le seul autre catalyseur exemplifié est l'alumine gamma.

La déshydratation d'alcools en $C_4$ sur solides acides s'accompagne généralement de l'isomérisation de position de l'alcène formé. Ces deux réactions sont en effet concomitantes, puisque l'isomérisation de position de la double liaison de l'alcène est aussi rapide que la réaction de déshydratation du monoalcool en $C_4$. Dans le cas de l'isobutanol, l'isobutène formé initialement se protonne facilement (formation d'un carbocation tertiaire) et peut ensuite subir des réactions secondaires, notamment de dimérisation, puis de cyclisation, risquant d'entraîner la formation de produits secondaires non désirés.

Kotsarenko et al., Kin. Katal. 24, 877 (1983) décrit cependant, dans le cas particulier de la déshydratation et de l'isomérisation squelettale simultanées de l'isobutanol sur solides non zéolithiques, un mécanisme dans lequel une espèce intermédiaire de type carbocation primaire formée par déshydratation sur site acide de l'alcool se réarrange via une réaction de méthyl shift pour former un carbocation secondaire et favoriser la formation de butènes linéaires. Les catalyseurs les plus performants sont des oxydes mixtes non organisés à base d'alumine et de silice, avec une teneur en alumine inférieure à 5%. La proportion maximale atteinte en n-butènes dans les butènes est de 32.7% à des températures comprises en 275 et 350°C.

Le document FR2733701 décrit un prétraitement sélectivant pour l'isomérisation des oléfines linéaires en isobutène par une mise en contact du catalyseur avec une ou plusieurs molécules hydrocarbonées, donc ne comprenant pas d'oxygène, contenant de 4 à 10 atomes de carbone à une pression comprise entre 0,1 et 1 MPa et à une température comprise entre 300 et 550°C. En résulte une augmentation de la sélectivité en isobutène et une diminution de la conversion des butènes. La présente invention vise au contraire à améliorer la sélectivité en butènes linéaires.

Cañizares et al., Applied Catalysis A : General 190 (2000) 93-105 divulguent dans un article traitant de l'isomérisation du n-butène sur zéolithe ferriérite modifiée par traitement au tétrachloride de silicone qu'une sélectivité élevée vers la production d'isobutène peut être obtenue par dépôt de coke sur le catalyseur. De manière surprenante, la mise en oeuvre du catalyseur selon l'invention permet d'obtenir une proportion en butènes linéaires plus importante, et donc une sélectivité en isobutènes plus faible, en procédant à un précokage du catalyseur.

La présente invention concerne un jeu de conditions opératoires, de choix de procédé et de préparation de catalyseur zéolithique qui permettent, par transformation d'un monoalcool primaire substitué en position 2 par un groupement alkyl en alcène, d'atteindre une proportion en alcènes linéaires dans la fraction alcènes bien supérieure à la valeur attendue à l'équilibre thermodynamique, avec une conversion totale de l'alcool et une sélectivité en alcènes totaux supérieure à 97%.

## OBJET ET INTÉRÊT DE L'INVENTION

L'invention concerne un procédé de transformation intégré thermiquement d'une charge comprenant d'un monoalcool primaire substitué en position 2 par un groupement alkyl sur un catalyseur pré-coké comprenant une zéolithe de type structural FER qui permet de produire un mélange d'alcènes riches en alcènes linéaires.

Le procédé selon l'invention permet d'obtenir, à l'issue de l'étape réactionnelle, un effluent comprenant une proportion en alcènes linéaires au-delà de celle attendue si l'on considère l'équilibre thermodynamique entre les alcènes à la température de sortie de réacteur, avec une excellente conversion et une très bonne sélectivité.

Le procédé selon l'invention permet également de limiter la dégradation thermique de la charge par la mise en oeuvre d'une chauffe appropriée, ainsi qu'une diminution globale de la quantité d'utilités chaudes et froides nécessaires.

## DESCRIPTION DÉTAILLÉE DE L'INVENTION

L'invention concerne un procédé de déshydratation isomérisante d'une charge comprenant de 40 à 100% poids d'alcool primaire substitué en position 2 par un groupement alkyl comprenant au moins les étapes suivantes :

a) Mise en pression de ladite charge puis préchauffe de la charge comprimée par échange de chaleur avec l'effluent de déshydratation issu de l'étape c) dans un échangeur de chaleur de manière à produire une charge préchauffée ;

b) Vaporisation de ladite charge préchauffée par mélange avec l'effluent diluant issu de l'étape f), le rapport des débits massiques effluent diluant sur charge préchauffée étant compris entre 5/95 et 60/40 ;

c) Déshydratation de l'effluent issu de l'étape b) dans au moins un réacteur de déshydratation opérant en phase gaz à une température moyenne pondérée comprise entre 250 et 375°C, à une pression comprise entre 0,2 MPa et 1 MPa et à une PPH comprise entre 1 et 18 h$^{-1}$, en présence d'un catalyseur comprenant une zéolithe de type structural FER, ledit catalyseur étant préalablement coké in-situ ou ex-situ, de manière à produire un effluent de déshydratation ;

d) Refroidissement dudit effluent de déshydratation par au moins trois échanges de chaleur indirects successifs avec au moins l'effluent eau issu de l'étape e), puis ladite charge comprimée de l'étape a), puis une utilité froide de manière à produire un effluent refroidi ;

e) Décantation dudit effluent refroidi en une phase aqueuse et une phase organique, une partie de ladite phase aqueuse étant purgée pour être traitée à l'extérieur dudit procédé de déshydratation et l'autre partie, formant l'effluent eau étant recyclée *via* l'étape f) ;

f) Recyclage de l'effluent eau issu de l'étape e) et vaporisation au moins partielle par échange de chaleur dans un échangeur de chaleur avec l'effluent de déshydratation issu de l'étape c), séparation de la fraction liquide éventuellement présente, puis compression et surchauffe de la fraction vapeur de manière à former un effluent diluant, ledit effluent diluant étant recyclé vers l'étape b) ;

g) Séparation de la phase organique extraite de l'étape e) dans au moins une colonne à distiller de manière à produire un effluent alcènes et un effluent hydrocarbures lourds.

### Charge

Conformément à l'invention, la charge traitée dans le procédé selon l'invention est une charge comprenant de 40 à 100% poids d'au moins un monoalcool primaire substitué en position 2 par un groupement alkyl. Ledit monoalcool est préférentiellement l'isobutanol ou le 2-methyl-1-butanol, pris seul ou en mélange, et très préférentiellement l'isobutanol. Dans la suite de l'exposé, on désigne par le terme « alcool primaire » le monoalcool primaire. Le terme alkyl désigne un composé hydrocarboné de formule générale $C_nH_{2n+1}$ où n est un entier compris entre 1 et 20, de préférence entre 1 et 10, de manière préférée entre 1 et 5.

Ladite charge peut provenir de procédés chimiques ou biochimiques, par exemple fermentaires. En particulier, cette charge peut être issue de procédés de fermentation de biomasse lignocellulosique.

Ladite charge comprend également avantageusement de 0 à 60% poids d'eau. Ladite charge peut également comprendre des impuretés de type minéral (telles que Na, Ca, P, Al, Si, K, SO$_4$) et de type organique (telles que du méthanol, de l'éthanol, du n-butanol, des aldéhydes, des cétones, et les acides correspondant, par exemple l'acide furanique, acétique, isobutyrique).

**Étape a) de préchauffe**

Conformément à l'invention, ladite charge comprenant un monoalcool primaire substitué en position 2 par un groupement alkyl est mise en pression dans une pompe puis préchauffée par échange de chaleur avec l'effluent de déshydratation issu de l'étape c) dans au moins un échangeur de chaleur de manière à produire une charge préchauffée.

Ladite pompe permet de monter la pression de ladite charge à une pression comprise entre 2 et 10 bar. L'échange de chaleur permet à la charge d'être chauffée à une température comprise entre 100 et 250°C, préférentiellement entre 100 et 150°C.

**Étape b) de vaporisation**

Conformément à l'invention, la charge préchauffée issue de l'étape a) est vaporisée par mélange avec l'effluent diluant issu de l'étape f).

L'effluent diluant issu de l'étape f) est constitué d'eau et d'impuretés dissoutes à hauteur de leur solubilité dans les conditions de l'étape de décantation e). Cet effluent est vaporisé, comprimé, et surchauffé dans l'étape d) de manière à apporter l'énergie suffisante à la vaporisation du mélange effluant diluant et charge préchauffé. La température de l'effluent diluant à la suite de sa surchauffe est comprise entre 400 et 650°C, préférentiellement entre 425 et 550°C.

Le rapport des débits massiques effluent diluant sur charge préchauffée est compris entre 5/95 et 60/40.

Le mélange charge préchauffée/effluent diluant est ensuite porté, dans un four, à une température comprise entre 250 et 375°C.

Lorsque l'on vise pour le fluide chauffé une température en sortie d'un équipement de chauffe, par exemple un four, la température de la surface d'échange est souvent bien plus élevée que la température visée, la différence pouvant être de l'ordre de 100°C. Au contact des parois, le fluide chauffé est donc soumis à des températures élevées. Ainsi, si l'on vise de 250 à 375°C en sortie de four, le mélange chauffé est exposé au contact des parois à des températures de l'ordre de 350 à 475°C. La chauffe de la charge en trois étapes comprenant une première étape de chauffe par échange de chaleur dans un échangeur de chaleur suivie d'une seconde étape de chauffe par mélange avec l'effluant diluant chaud et une troisième étape de chauffe dans un four permet d'éviter que le monoalcool primaire substitué en position 2 par un groupement alkyl ne se retrouve exposé concentré, c'est-à-dire dans un mélange comprenant plus de 95% poids de monoalcool primaire substitué en position 2 par un groupement alkyl, à des températures supérieures à 280°C. Le monoalcool primaire substitué en position 2 par un groupement alkyl est ainsi protégé des risques de dégradation thermique, ce qui améliore le rendement global du procédé.

**Étape c) de déshydratation**

Conformément à l'invention, l'effluent issu de l'étape b) alimente ensuite une étape de déshydratation.

L'étape de déshydratation comprend au moins un réacteur de déshydratation. Lorsque cette étape comprend plus d'un réacteur, la température à l'entrée de chacun des réacteurs est ajustée à une valeur comprise entre 250 et 375°C par un moyen de chauffe, la réaction de déshydratation isomérisante étant endothermique, et chaque réacteur est opéré dans des conditions identiques. Ainsi, dans la suite de l'exposé, le terme « le réacteur » désigne aussi bien le réacteur de ladite étape c), lorsque celle-ci ne comprend qu'un réacteur, que chacun des réacteurs de ladite étape c), lorsque celle-ci comprend plus d'un réacteur.

Le réacteur est opéré en phase gaz, à une température moyenne pondérée comprise entre 250 et 375°C, à une pression comprise entre 0,2 MPa et 1 MPa, à une PPH comprise entre 1 et 18 h$^{-1}$, en présence d'un catalyseur comprenant une zéolithe comprenant au moins une série de canaux dont l'ouverture est définie par un anneau à 8 atomes d'oxygène (8MR). Ledit catalyseur est disposé dans un ou plusieurs lits fixes, lesquels peuvent être opérés en écoulement ascendant, descendant ou radial.

Par PPH, on entend « Poids par Poids par Heure », c'est-à-dire le débit massique d'alcool primaire substitué en position 2 par un groupement alkyl dans la charge en entrée de réacteur divisé par la masse de catalyseur dans ledit réacteur. Cette notion est également parfois désignée sous son acronyme anglais de WHSV, ou « Weight Hourly Space Velocity ».

Par température moyenne pondérée, on entend la moyenne de la température dans le lit catalytique calculée le long de l'axe d'écoulement dans ledit lit. Soit un lit de longueur L et de surface S, le mélange réactif s'écoulant le long de l'axe longitudinal $x$ de ce lit, l'entrée dans le lit catalytique formant l'origine de l'axe ($x$=0), la température moyenne pondérée s'exprime selon :

$$\text{TMP} = \frac{1}{L}\int_0^L T(x)dx$$

Conformément à l'invention, ledit catalyseur est pré-coké in-situ ou ex-situ avec une charge comprenant de l'alcool primaire substitué en position 2 par un groupement alkyl à une pression partielle en ledit alcool primaire strictement supérieure à celle de la charge du procédé, ou bien avec ledit alcool primaire pur. Par ledit alcool primaire pur, on entend que ledit alcool primaire substitué en position 2 par un groupement alkyl comprend moins de 1% masse de composés autre que ledit alcool primaire, de préférence moins de 1000 ppm, de manière préférée moins de 100 ppm, très préférentiellement moins de 10 ppm et de manière très préférée qu'il ne comprend pas d'autres composés détectables. Dans un autre arrangement, le pré-cokage pourra être réalisé avec l'effluent hydrocarbures lourds issu de l'étape g) de séparation .

Dans un premier mode de réalisation de l'invention, le pré-cokage est réalisé à une température moyenne pondérée strictement supérieure à la température opératoire de la réaction et comprise entre 250 et 450°C, avantageusement entre 300 et 450°C, et très avantageusement entre 400 et 450°C, une pression comprise entre 0,1 et 3 MPa, avantageusement entre 0,1 et 0,5MPa, et une PPH comprise entre 0,1 et 10 h⁻¹, avantageusement entre 0,1 et 3 h⁻¹.

Dans un second mode de réalisation, le pré-cokage est réalisé à une température moyenne pondérée strictement inférieure à la température moyenne pondérée opératoire de la réaction, et comprise entre 200 et 350°C, préférentiellement entre 225 et 325°C, une pression strictement supérieure à la pression opératoire de la réaction et comprise entre 0,1 et 3 MPa, avantageusement entre 1,1 et 3 MPa, préférentiellement comprise entre 1,2 et 3 MPa, et une PPH comprise entre 0,1 et 10 h⁻¹, préférentiellement entre 0,1 et 3 h⁻¹. Ce mode de réalisation permet en particulier d'éviter le dépôt de coke lourd sur le catalyseur.

Dans ces deux modes de réalisation, le pré-cokage est réalisée pendant une durée de 1 à 30 h, de préférence de 2 à 24 h. Ce pré-cokage, effectué soit à une température moyenne pondérée strictement supérieure à la température moyenne pondérée d'opération de la réaction de déshydration isomérisante, soit à une température moyenne pondérée strictement inférieure et une pression strictement supérieure aux température moyenne pondérée et pression d'opération de la réaction de déshydration isomérisante permet d'améliorer significativement la sélectivité du catalyseur en alcènes linéaires, au-delà de celle attendue si l'on considère l'équilibre thermodynamique entre les alcènes à la température de sortie de réacteur. Il est effectué préalablement à la mise en oeuvre dudit catalyseur pour réaliser la réaction de déshydration isomérisante de la charge comprenant de l'alcool primaire substitué en position 2 par un groupement alkyl.

La conversion baisse régulièrement au cours de l'opération. Le catalyseur est régulièrement régénéré par combustion du coke en présence d'oxygène dilué dans de l'azote, la dilution étant ajustée de manière à maintenir une température au sein du lit catalytique comprise entre 400 et 600°C. Cette régénération est suivie d'un pré-cokage selon l'invention avant la remise en opération du catalyseur.

La durée de pré-cokage est considérée comme suffisante si, lors de l'opération du réacteur de manière à réaliser la réaction de déshydratation isomérisante de ladite charge du procédé, la sélectivité en C₅⁺ est inférieure à 1% pour une conversion dudit monoalcool primaire substitué en position 2 par un groupement alkyl compris dans ladite charge du procédé supérieure à 97%. La sélectivité en C₅⁺ est définie comme le ratio de la différence entre le débit massique d'élément carbone compris dans les hydrocarbures ayant au moins 5 atomes de carbone dans l'effluent de déshydratation et le débit massique d'élément carbone compris dans les hydrocarbures ayant au moins 5 atomes de carbone dans l'effluent issu de l'étape b), sur la différence entre le débit massique d'élément carbone compris dans l'alcool primaire substitué en position 2 par un groupement alkyl compris dans l'effluent issu de l'étape b) et le débit massique d'élément carbone compris dans l'alcool primaire substitué en position 2 par un groupement alkyl compris dans l'effluent de déshydratation.

Ladite zéolithe est choisie parmis les zéolithes de type structural FER et avantageusement parmis les zéolithes ferrierite, FU-9, ISI-6, NU-23, ZSM-35 et ZSM-57, prise seule ou en mélange. Ladite zéolithe est plus avantageusement la ferrierite.

Ledit catalyseur comprend ladite zéolithe mise en forme dans une matrice constituée d'un liant à caractère inerte. En effet, ladite zéolithe ne peut être utilisée industriellement sous forme de poudre. Le liant permet de conférer au solide final une résistance accrue en présence d'eau.

Le rapport massique liant sur zéolithe dudit catalyseur est compris entre 50/50 et 10/90.

De manière très avantageuse, ledit catalyseur est constitué de zéolithe ferrierite et de liant silicique.

Ledit catalyseur utilisé dans le procédé selon l'invention est avantageusement préparé selon un procédé de préparation comprenant au moins les étapes suivantes :

1) une étape de mélange d'au moins une poudre de zéolithe sous forme protonique ou ammonium avec au moins une poudre d'au moins un liant, par exemple une poudre de silice amorphe, ce qui contribue à contrôler la porosité du solide final, et au moins un solvant en présence d'un agent peptisant ;

2) une étape d'ajout d'un solvant, avantageusement de l'eau, afin de moduler la perte au feu de la pâte et d'obtenir les propriétés texturales souhaitées pour le solide final ;

3) une étape de mise en forme du mélange pâteux obtenu à l'issue de l'étape 2), par exemple par extrusion ;

4) une étape de séchage du matériau mis en forme obtenu à l'issue de l'étape 3), avantageusement à une température comprise entre 50 et 200°C, préférentiellement entre 80 et 150°C, avantageusement pendant une durée comprise entre 1 et 24 h, et avantageusement sous air ;

5) une étape optionnelle de calcination à une température allant de 400 à 800°C pendant une période allant de 2 à 12 h ;

6) une étape optionnelle de traitement thermique, telle qu'un étuvage à une température comprise entre 500-700°C sous air humide, c'est-à-dire comprenant de 6 à 50% volume d'eau dans l'air.

Le liant utilisé dans l'étape 1 pourra être choisi parmi les liants bien connus de l'Homme du métier, et plus particulièrement parmi ceux présentant une « inertie » vis-à-vis des conditions opératoires et notamment de la présence d'eau dans le procédé. Ainsi, on pourra avantageusement utiliser un liant silicique, un liant aluminophosphate ou une argile.

Une source de liant silicique peut être une silice de précipitation ou une silice issue de sous-produits comme les cendres volantes, par exemple les particules silico-alumineuses ou silico-calciques, et les fumées de silice. On pourra avantageusement utiliser une silice colloïdale, se présentant par exemple sous la forme d'une suspension stabilisée, telles que par exemple des produits commerciaux tels que le Ludox® ou les Klebosol®.

La poudre de silice amorphe avantageusement utilisée dans l'étape 1) du procédé de préparation du catalyseur utilisé selon l'invention présente de manière préférée une granulométrie adaptée à 1,4 ou 1,8 $\mu$m.

Les poudres sont avantageusement malaxées en présence d'un solvant (étape 2), de préférence de l'eau dans lequel un agent peptisant peut avantageusement être dissout afin d'obtenir une meilleure dispersion du liant. La consistance de la pâte est ajustée par le biais de la quantité de solvant.

L'agent peptisant utilisé lors de cette étape peut avantageusement être un acide, une base organique ou inorganique tel que l'acide acétique, l'acide chlorhydrique, l'acide sulfurique, l'acide formique, l'acide citrique et l'acide nitrique, seul ou en mélange, la soude, la potasse, l'ammoniaque, une amine, un composé à ammonium quaternaire, choisi parmi les alkyl-éthanol amines ou les alkyl- amines éthoxylées, l'hydroxyde de tétraéthylammonium et le tétraméthylammonium.

Le protocole de mise en forme du solide ne doit pas modifier l'accès à la partie active de celui-ci : la zéolithe, et doit faciliter la diffusion des réactifs dans le solide. Le catalyseur mis en forme peut revêtir toute forme connue de l'Homme du métier, telle que tablette, granule, extrudé mono ou polylobé, sphère.

Ledit catalyseur est micro/méso/macroporeux.

**Étape d) de refroidissement**

L'effluent issu du dernier réacteur de l'étape c) est à une température d'environ 250°C, en phase vapeur.

Conformément à l'invention, l'effluent de l'étape c) est refroidi par au moins trois échanges de chaleur successifs, avec au moins l'effluent eau issu de l'étape e), puis ladite charge comprimée de l'étape a), puis une utilité froide de manière à produire un effluent refroidi. Les échanges de chaleur sont réalisés dans des échangeurs de chaleur dont la technologie est bien connue de l'Homme du métier (échangeurs à plaque, échangeurs tube-calandre ou autres systèmes adaptés).

L'effluent de l'étape c) est refroidi et partiellement condensé à l'issue du premier échange de chaleur avec l'effluent eau issu de l'étape e). Le refroidissement est poursuivi avec le deuxième échange de chaleur avec la charge de l'étape a) mise en pression, puis avec le troisième échange avec l'utilité froide. À l'issue du troisième échange de chaleur, l'effluent de l'étape c) est totalement condensé, et à une température inférieure à 50°C. Il est ensuite dirigé vers l'étape e) de décantation.

Les échanges de chaleurs sont réalisés dans des échangeurs de chaleur connus de l'Homme du métier, pouvant comporter plusieurs calandres et, éventuellement, un circuit d'évitement contournant une ou plusieurs de ces calandres afin de régler la température des flux en sortie. L'utilité froide peut être de l'air, de l'eau de refroidissement, et/ou tout autre fluide disponible permettant à l'effluent issu de l'étape c) d'atteindre une température inférieure à 50°C.

**Étape e) de décantation**

Conformément à l'invention, l'effluent de l'étape c), refroidi et condensé dans l'étape d), alimente une étape e) de décantation. Une démixtion a lieu et l'étape e) de décantation permet de séparer une phase aqueuse et une phase organique.

La phase aqueuse comprend plus de 90% poids d'eau, préférentiellement plus de 95% poids d'eau. La teneur en eau de la phase aqueuse dépend fortement de la présence d'espèces ne réagissant pas dans l'étape c) de déshydratation et solubles dans l'eau, qui se retrouvent toutes dans ladite phase aqueuse comme par exemple le méthanol, l'éthanol, l'acétaldéhyde, l'acétone et les acides correspondant. Elle comprend également les composés présents dans la phase organique à hauteur de leur solubilité, principalement quelques ppm de composés oxygénés et environ 5 ppm d'alcool primaire substitué en position 2 par un groupement alkyl.

La phase organique contient l'alcool primaire substitué en position 2 par un groupement alkyl n'ayant pas réagi, ainsi que les alcènes et les produits lourds produits lors de l'étape c) de déshydratation.

La phase aqueuse est soutirée en vue d'être recyclée. Une partie de cette phase est purgée afin de maintenir à une valeur de quelques ppm la teneur en composés lourds dans le recyclage. Cette partie purgée peut être traitée à l'extérieur du procédé. La partie non purgée, qui forme l'effluent eau, est recyclée via l'étape f) de recyclage. La phase organique est traitée dans l'étape g) de séparation afin de produire au moins un effluent alcènes.

La fraction purgée représente de 0 à 30% de la phase aqueuse extraite, avantageusement de 5 à 20% de cette phase.

## Étape f) de recyclage

L'effluent eau soutiré dans l'étape e) de décantation est détendu. Par détendu, on entend que sa pression est abaissée. La détente détermine le point bas de pression de la boucle de recyclage d'eau.

L'effluent eau détendu est réchauffé par échange de chaleur dans un échangeur de chaleur avec l'effluent de l'étape c) de déshydratation, au cours duquel il est au moins partiellement vaporisé. Par partiellement vaporisé, on entend qu'au moins 90% poids de l'effluent eau détendu est vaporisé.

La pression de détente de l'effluent eau est ajustée de manière à récupérer le maximum de chaleur lors de l'échange de chaleur avec l'effluent de l'étape c), c'est-à-dire que la vaporisation à l'issue de l'échange de chaleur soit d'au moins 90% poids de l'effluent eau, mais qu'elle ne soit pas totale.

La fraction liquide éventuellement présente est séparée dans un ballon séparateur destiné à protéger le compresseur de toute présence de liquide (« K.O. Drum » selon la terminologie de anglo-saxonne). La fraction vapeur, débarrassée de la fraction liquide, est comprimée dans un compresseur. Le compresseur fixe le point de pression haut de la boucle de recyclage.

L'ajustement du point haut et du point bas de pression du procédé permet d'une part d'assurer une pression suffisante dans l'étape c) de déshydratation, mais également d'assurer une récupération maximale de la chaleur de l'effluent de déshydratation en vaporisant au moins 90% de l'effluent eau. Cet ajustement est également effectué de telle manière que, tout en récupérant le maximum de chaleur de l'effluent de déshydratation, la température de la fraction vapeur de l'effluent eau soit telle qu'à l'issue de la compression, la température n'excède pas 300°C afin de ne pas endommager les équipements.

La fraction vapeur comprimée, à une température qui n'excède pas 300°C, est surchauffée dans un échangeur de chaleur, par exemple un four, à une température comprise entre 400 et 650°C.

Dans un arrangement préféré, un seul four est utilisé pour la chauffe du mélange charge préchauffée/effluent diluant au cours de l'étape b) et pour la surchauffe de la fraction vapeur comprimé, cette dernière étant surchauffée dans la partie chaude du four, tandis que ledit mélange est mis en température dans la partie froide.

La fraction vapeur comprimée et surchauffée forme l'effluent diluant. Cet effluent est ensuite mélangé à la charge préchauffée issue de l'étape a) afin d'assurer son évaporation.

## Étape g) de séparation

Conformément à l'invention, la phase organique extraite de l'étape e) est traitée dans une étape de séparation de manière à produire un effluent alcènes et un effluent hydrocarbures lourds.

Ladite étape de séparation comprend au moins une colonne à distiller. Ladite phase organique est séparée par distillation et l'on récupère en tête un distillat comprenant les alcènes et en fond un résidu comprenant les produits lourds ainsi que l'alcool primaire substitué en position 2 par un groupement alkyl non réagi.

Cette colonne possède de 5 à 20 plateaux théoriques, avantageusement de 5 à 15. La colonne est opérée à une pression comprise entre 0,5 et 1 MPa, avec une température de tête comprise entre 50 et 90°C, et une température de fond comprise entre 100 et 150°C.

Le distillat est avantageusement dirigé vers un sécheur, nécessaire pour ajuster la teneur en eau en fonction des applications aval.

L'alcool primaire substitué en position 2 par un groupement alkyl non converti, compris dans le résidu, peut avantageusement être recyclé en mélange avec la charge du procédé selon l'invention.

# EXEMPLES

### Exemple 1 (non-conforme) : chauffe de l'isobutanol :

*Cet exemple illustre la décomposition thermique de l'isobutanol pur lorsqu'il est porté à haute température.*

De l'isobutanol pur, c'est-à-dire en particulier exempt d'eau, est vaporisé dans un four dans lequel un lit de carborundum inerte (SiC) a été installé afin de favoriser l'échange thermique. Il est exposé à plusieurs températures, à une pression de 0,1 MPa. La PPH est de 1 h$^{-1}$. La composition de l'effluent est analysée. Les résultats sont présentés Tableau 1.

1,5% de l'isobutanol est transformé à 300°C en produits de déshydrogénation, de déshydratation et d'isomérisation de l'isobutène, et d'autres produits non identifiés. 4.5% de l'isobutanol est transformé à 400°C.

**Tableau 1 - Analyse de l'effluent en sortie de zone de chauffe**

| %molaire | charge | 300°C | 350°C | 400°C | 450°C |
|---|---|---|---|---|---|
| Isobutanol | 99.35 | 98.93 | 98.99 | 95.51 | 86.22 |
| 2-butanone | 0.55 | 0.61 | 0.29 | 0.42 | 0.45 |
| 1-butanol | 0.07 | 0.07 | 0.07 | 0.06 | 0.05 |
| 2-butanol | | 0.02 | 0.02 | 0.09 | 0.13 |
| Isobutyraldéhyde | | 0.1-0.31 | 0.43 | 2.59 | 8.24 |
| Isobutène + 1-butène | | 0.04 | 0.18 | 1.05 | 3.39 |
| 2-butènes | | | 0.02 | 0.12 | 0.42 |
| propane | 0.01 | 0.01 | | 0.07 | 0.6 |
| méthane | | | | 0.02 | 0.14 |

Le rapport de surface A(H$_2$)/A(N$_2$) et le ratio H$_2$ en sortie/iC$_4$OH en entrée permettent de mettre en évidence l'occurrence de la réaction de déshydrogénation et l'impact de la température sur celle-ci.

### Description de l'unité de test catalytique et des catalyseurs utilisés pour les exemples 2 à 5

Les exemples 2 à 5 sont réalisés sur une unité de test comprenant un lit fixe fonctionnant en mode « down flow », c'est-à-dire en écoulement descendant. Le catalyseur est chargé sous forme d'extrudés de longueur 3mm dans un réacteur inox 316L de diamètre interne de 13mm. Le catalyseur est ensuite activé à 450°C sous 6l/h d'air pendant un palier d'une heure, après une montée en température de 10°C/min, la température est ensuite abaissée à la température de test sous 6l/h d'azote afin d'éliminer l'air présent dans le système avant injection de la charge.

La charge est vaporisée dans les lignes chauffées à 150-180°C en amont du réacteur puis injectée dans le réacteur catalytique. Chaque condition de température et de pph est maintenue un minimum de 24h (la durée minimale d'un test est de 96h). Le catalyseur est régénéré plusieurs fois sous air à 500°C.

Le catalyseur A est préparé par comalaxage de 80% de ferrierite présentant un ratio atomique Si/Al de 20 et de 20% de liant silicique. Le solide a été extrudé, séché à 80°C pendant 12h puis calciné sous air sec pendant 2 h à 600°C. Le catalyseur A obtenu a une surface spécifique SBET de 321 m$^2$/g, un volume mésoporeux de 0,11 cc/g, macroporeux de 0,35 cc/g et microporeux de 0,11 cc/g.

### Exemple 2

*Déshydratation d'une charge isobutanol/eau en rapport massique 95/5 sur le catalyseur A et sur un catalyseur B. Cet exemple montre l'intérêt du pré-cokage.*

Le catalyseur B est préparé par comalaxage de 80% de ferrierite présentant un ratio atomique Si/Al de 10 et de 20% de liant silicique. Le solide a été extrudé, séché à 80°C pendant 12h puis calciné sous air sec pendant 2 h à 600°C. Le catalyseur B obtenu a une surface spécifique SBET de 320 m$^2$/g, un volume mésoporeux de 0,09 cc/g, macroporeux de 0,19 cc/g et microporeux de 0,11 cc/g.

Les catalyseurs ont dans un premier temps été testés sans pré-cokage à 0,2 Mpa, pph 3h$^{-1}$, et une température moyenne pondérée (TMP) de 350°C.

Les mêmes catalyseurs ont été au préalable pré-cokés sous charge isobutanol + eau à 1,2 Mpa, TMP de 350°C

pph 3 h$^{-1}$ pendant 6 h puis testés à 0,2 Mpa, pph 3 h$^{-1}$, TMP de 350°C. Les performances catalytiques des catalyseurs pré-cokés ou non ont été mesurées. Les résultats sont donnés pour les deux catalyseurs A et B pré-cokés ou non dans le tableau ci-dessous. Les données ci-dessous ont été obtenues après 6h sous charge. Par C$_3$$^=$ on entend propène, par C$_4$$^=$ on entend butènes, et par C$_5$$^+$ on entend hydrocarbures ayant au moins 5 atomes de carbone.

| Description | | Sélectivité isobutène (%) | Rendement en butènes linéaires (%pds) * | sel C3= (%) | Sel C5+ (%) | Sel C4= (%) | Conversion isobutanol (%) | % butènes linéaires dans les C4 oléfines |
|---|---|---|---|---|---|---|---|---|
| Catalyseur A | Non pré-coké | 24.5 | 72.4 | 0.05 | 2.4 | 96.9 | 100.0 | 74.7 |
| | pré-coké | 18.7 | 80.6 | 0.0 | 0.4 | 99.5 | 99.9 | 81.2 |
| Catalyseur B | Non pré-coké | 24.9 | 50.1 | 2.03 | 19.7 | 75 | 100 | 50.1 |
| | pré-coké | 18.2 | 80.5 | 0.05 | 1.0 | 98 | 99.7 | 80.4 |
| * calcul effectué sur une base carbone, sans tenir compte de la fraction eau éliminée | | | | | | | | |

Sur un catalyseur pré-coké sous charge, la conversion de l'alcool reste totale, la sélectivité en butènes totaux augmente grâce à un abaissement important de la sélectivité en produits C$_5$$^+$ et C$_3$$^=$ dont il est couramment admis qu'ils sont issus de la dimérisation-craquage des butènes en propylène plus C$_5$. Ainsi, pour une zéolithe quel que soit son Si/Al de départ et sa sélectivité initiale, le pré-cokage dans des conditions choisies permet d'obtenir un catalyseur beaucoup plus sélectif pour la transformation de l'isobutanol en butènes linéaires en limitant la formation de produits plus lourds que les C$_4$. Ce qui permet d'amener la sélectivité d'un catalyseur initialement peu sélectif à celle d'un catalyseur plus sélectif.

## Exemple 3

*Déshydratation d'une charge isobutanol/eau en rapport massique 95/5 sur le catalyseur A. Cet exemple montre que la ferrierite catalyse une réaction dans laquelle les oléfines linéaires sont un produit primaire de la réaction.*
Les conditions opératoires et les résultats catalytiques sont donnés ci-dessous. Le réacteur est maintenu à une pression opératoire de 0,2 MPa. Le catalyseur n'est pas pré-coké. Les données sont obtenues en moyennant les valeurs sur 24h.

| T (°C) | pph (h$^{-1}$) | Sel isobutène (%) | Sel C5+ (%) | Sel C4= (%) | Conversion alcool (%) | butènes linéaires dans les C4 oléfines (%) |
|---|---|---|---|---|---|---|
| 300 | 21 | 23.65 | 2.2 | 97.4 | 99.1 | 75.7 |
| 300 | 12 | 24.16 | 2.4 | 97.3 | 99.8 | 75.2 |
| 300 | 5 | 25.24 | 2.6 | 97.0 | 100.0 | 74.0 |
| 300 | 3 | 27.43 | 3.2 | 96.3 | 100.0 | 71.5 |
| 300 | 1 | 32.34 | 5.0 | 94.1 | 100.0 | 65.6 |
| * les autres produits secondaires permettant de faire boucler la somme des sélectivités à 100% sont principalement de l'isobutyraldéhyde, de l'isobutane et du propane/propylène. | | | | | | |

Une augmentation de la pph entraîne une diminution de la sélectivité en isobutène et une augmentation de la sélectivité en butènes linéaires. Ceci indique que l'isobutène n'est pas un produit primaire de la réaction, qui subirait une isomérisation squelettale pour former des butènes linéaires. Au contraire, ce sont les oléfines linéaires qui sont les produits primaires de la réaction. Le catalyseur selon l'invention permet d'obtenir une proportion de butènes linéaires dans les butènes totaux largement supérieure à la valeur attendue à l'équilibre thermodynamique qui est comprise entre 45 et 55%, ainsi qu'une sélectivité en butènes supérieure à 96% excepté pour la pph 1h$^{-1}$. La déshydratation de l'isobutanol est totale quelle que soit les conditions opératoires, excepté pour la pph la plus élevée.

## Exemple 4

*Déshydratation d'une charge 1-butanol/eau en rapport massique 95/5 et d'une charge isobutanol/eau en rapport massique 95/5 sur la catalyseur A. Cet exemple montre que le catalyseur selon l'invention ne favorise pas l'isomérisation squelettale des butènes linéaires.*

Un essai a été réalisé en utilisant une charge 1-butanol diluée avec 5% d'eau ou avec une charge isobutanol diluée avec 5% d'eau que l'on fait réagir en présence du catalyseur A sous pression opératoire de 0,2 MPa.

Le catalyseur A est pré-coké avec la charge à 1,2 Mpa, 350°C, pph 3h$^{-1}$ pendant 6h. Il est donc utilisé conformément à l'invention.

| TMP (°C) | pph (h$^{-1}$) | Sel isobutène (%) | Sel C5+ (%) | Sel C4= (%) | Conversion alcool (%) | % butènes linéaires dans les C4 oléfines |
|---|---|---|---|---|---|---|
| | | | Charge 1 butanol/eau | | | |
| 350 | 6 | 12.6 | 2.6 | 97.0 | 99.9 | 84.1 |
| 375 | 6 | 15.6 | 2.2 | 97.3 | 100.0 | 81.7 |
| 375 | 12 | 10.3 | 1.9 | 97.9 | 99.9 | 87.6 |
| 350 | 3 | 7.9 | 1.2 | 98.6 | 100.0 | 90.6 |
| | | | Charge isobutanol/eau | | | |
| 350 | 6 | 19.8 | 0.6 | 99.1 | 99.1 | 80.1 |
| 375 | 6 | 20.9 | 0.4 | 99.4 | 99.5 | 78.9 |
| 375 | 12 | 20.4 | 0.3 | 99.5 | 99.0 | 79.5 |
| 350 | 3 | 18.7 | 0.4 | 99.5 | 99.7 | 81.2 |

On notera que le test à 350°C et pph 6 avec une charge isobutanol/eau sur le catalyseur utilisé conformément à l'invention peut se comparer à l'exemple 1 de WO 2011/113834, deuxième colonne. En effet, la WHSV dans ce document est calculée sur la masse de ferriérite. La pph est, dans notre tableau, calculé sur la base du catalyseur, lequel contient 20% de liant et 80% de ferriérite. Par conséquent, une pph de 6 correspond à une WHSV selon WO 2011/113834 de 6/0,8=7,5. On observe une meilleure sélectivité en $C_4^{=}$ et une proportion en butènes linéaires dans les $C_4$ oléfines bien supérieure avec le catalyseur mis en oeuvre selon l'invention.

Le catalyseur utilisé selon l'invention permet d'obtenir une proportion de butènes linéaires dans les butènes totaux largement supérieure à la valeur attendue à l'équilibre thermodynamique qui est comprise entre 45 et 55%, ainsi qu'une sélectivité en butènes supérieure à 97%. La déshydratation de l'isobutanol est totale quelles que soient les conditions opératoires. La sélectivité en butènes linéaires est quasi identique que l'on parte de la charge butanol ou isobutanol. Le catalyseur ne favorise pas l'isomérisation squelettale des butènes linéaires en isobutène, même en allongeant le temps de contact (en abaissant la pph). Ceci indique que le catalyseur utilisé selon l'invention à base de ferrierite a une sélectivité de forme particulière favorisant la formation des butènes linéaires et non de l'isobutène à partir de l'isobutanol ou du 1-butanol dans les conditions opératoires choisies.

## Exemple 5 (comparatif)

*Cet exemple montre l'intérêt des zéolithes de type structural FER présentant des canaux 8 et 10MR sur les zéolithes de type structural TON et MTT présentant uniquement des canaux 10MR.*

Quatre zéolithes contenant des canaux 10MR de type structural FER (ferrierite bidimensionnelles 8-10MR), TON (Nu-10 monodimensionnelle 10MR) et MTT (ZSM-23 monodimensionnelle 10MR) ont été comparées. Ces zéolithes ont été testées sous forme de poudre de granulométrie 400-500 $\mu$m à TMP de 350°C et à pph 3 h$^{-1}$, 0.2 MPa.

Les catalyseurs sont pré-cokés avec la charge à 1,2 Mpa, 350°C, pph 3h$^{-1}$ pendant 6h. Les catalyseurs A et B sont donc utilisés conformément à l'invention.

| Réf Catalyseur | pph (h⁻¹) | Sel C3= (%) | Sel C5+ (%) | Sel C4= (%) | Conversion isobutanol (%) | % butènes linéaires dans les C4 oléfines |
|---|---|---|---|---|---|---|
| B | 3 | 0.05 | 1.0 | 98 | 99.7 | 80.4 |
| A | 3 | 0.0 | 0.4 | 99.5 | 99.9 | 81.2 |
| Nu-10 Si/Al 30 | 3 | 1.1 | 14.8 | 82.7 | 100.0 | 57.8 |
| ZSM-23 Si/Al 22 | 3 | 8.2 | 59.5 | 16.5 | 99.3 | 44.5 |

Seules les ferrierite permettent d'atteindre des proportions en butènes linéaires dans la coupe C4 oléfines supérieures à la composition à l'équilibre thermodynamique ainsi qu'une sélectivité en butènes élevée. Sur toutes les zéolithes, la conversion de l'alcool est totale. La zéolithes ZSM-23 présente par contre une sélectivité très dégradée et favorisent la formation de C5+ et de C3=. La zéolithe Nu-10 est légèrement plus sélective mais favorise encore fortement la formation de produits secondaires.

**Exemple 6**

*Déshydratation d'une charge isobutanol/eau en rapport massique 95/5 sur le catalyseur A. Cet exemple montre que la ferrierite catalyse une réaction dans laquelle les oléfines linéaires sont un produit primaire de la réaction. Cet exemple, comparativement à l'exemple 3 montre les effets bénéfique du précokage selon l'invention à basse température.*
Les conditions opératoires et les résultats catalytiques sont donnés ci-dessous. Le réacteur est maintenu à une pression opératoire de 0,2 MPa. Le catalyseur est pré-coké. Les données sont obtenues en moyennant les valeurs sur 24h.

| T (°C) | pph (h⁻¹) | Sel isobutène (%) | Sel C5+ (%) | Sel C4= (%) | Conversion alcool (%) | butènes linéaires dans les C4 oléfines (%) |
|---|---|---|---|---|---|---|
| 300 | 21 | 22.51 | 0.2 | 99.7 | 99.0 | 85.1 |
| 300 | 12 | 23.04 | 0.3 | 99.6 | 99.5 | 85.0 |
| 300 | 5 | 24.12 | 0.4 | 99.5 | 99.6 | 84.6 |
| 300 | 3 | 26.31 | 0.4 | 99.5 | 99.8 | 84.5 |
| 300 | 1 | 30.14 | 0.6 | 98.9 | 100.0 | 82.8 |
| * les autres produits secondaires permettant de faire boucler la somme des sélectivités à 100% sont principalement de l'isobutyraldéhyde, de l'isobutane et du propane/propylène. | | | | | | |

Comme dans l'exemple 3, une augmentation de la pph entraîne une diminution de la sélectivité en isobutène et une augmentation de la sélectivité en butènes linéaires. Ceci indique que l'isobutène n'est pas un produit primaire de la réaction, qui subirait une isomérisation squelettale pour former des butènes linéaires. Le catalyseur selon l'invention permet d'obtenir une proportion de butènes linéaires dans les butènes totaux largement supérieure à la valeur attendue à l'équilibre thermodynamique qui est comprise entre 45 et 55%, ainsi qu'une sélectivité en butènes, dans le cas du catalyseur précoké supérieure à 99% excepté pour la pph 1h⁻¹. La déshydratation de l'isobutanol est totale quelle que soit les conditions opératoires, excepté pour la pph la plus élevée.
Comparativement à l'exemple 3, on observe en raison du précokage une amélioration de la sélectivité globale en oléfines C4= produite significative. Il y a en outre un gain plus léger sur le taux d'isomérisation (baisse de l'isobutène résiduel), au prix d'une légère perte d'activité.

**Revendications**

1.  Procédé de déshydratation isomérisante d'une charge comprenant de 40 à 100% poids d'alcool primaire substitué en position 2 par un groupement alkyl comprenant au moins les étapes suivantes :

a) Mise en pression de ladite charge puis préchauffe de la charge comprimée par échange de chaleur avec l'effluent de déshydratation issu de l'étape c) dans un échangeur de chaleur de manière à produire une charge préchauffée ;

b) Vaporisation de ladite charge préchauffée par mélange avec l'effluent diluant issu de l'étape f), le rapport des débits massiques effluent diluant sur charge préchauffée étant compris entre 5/95 et 60/40 ;

c) Déshydratation de l'effluent issu de l'étape b) dans au moins un réacteur de déshydratation opérant en phase gaz à une température moyenne pondérée comprise entre 250 et 375°C, à une pression comprise entre 0,2 MPa et 1 MPa et à une PPH comprise entre 1 et 18 $h^{-1}$, en présence d'un catalyseur comprenant une zéolithe de type structural FER, ledit catalyseur étant préalablement coké in-situ ou ex-situ, de manière à produire un effluent de déshydratation ;

d) Refroidissement dudit effluent de déshydratation par au moins trois échanges de chaleur successifs avec au moins l'effluent eau issu de l'étape e), puis ladite charge comprimée de l'étape a), puis une utilité froide de manière à produire un effluent refroidi ;

e) Décantation dudit effluent refroidi en une phase aqueuse et une phase organique, une partie de ladite phase aqueuse étant purgée pour être traitée à l'extérieur dudit procédé de déshydratation et l'autre partie, formant l'effluent eau étant recyclée *via* l'étape f) ;

f) Recyclage de l'effluent eau issu de l'étape e) et vaporisation au moins partielle par échange de chaleur dans un échangeur de chaleur avec l'effluent de déshydratation issu de l'étape c), séparation de la fraction liquide éventuellement présente, puis compression et surchauffe de la fraction vapeur de manière à former un effluent diluant, ledit effluent diluant étant recyclé vers l'étape b) ;

g) Séparation de la phase organique extraite de l'étape e) dans au moins une colonne à distiller de manière à produire un effluent alcènes et un effluent hydrocarbures lourds.

**2.** Procédé selon la revendication 1 dans lequel ledit catalyseur de ladite étape c) est pré-coké avec une charge comprenant de l'alcool primaire substitué en position 2 par un groupement alkyl à une pression partielle en ledit alcool primaire strictement supérieure à celle de la charge dudit procédé selon la revendication 1, à une température moyenne pondérée strictement supérieure à la température opératoire et comprise entre 250 et 450°C, une pression comprise entre 0,1 et 3 MPa, une PPH comprise entre 0,1 et 10 $h^{-1}$ et une durée comprise entre 1 et 30 h.

**3.** Procédé selon la revendication 1 dans lequel ledit catalyseur de ladite étape c) est pré-coké avec une charge comprenant de l'alcool primaire substitué en position 2 par un groupement alkyl à une pression partielle en ledit alcool primaire strictement supérieure à celle de la charge dudit procédé selon la revendication 1, à une température moyenne pondérée strictement inférieure à la température opératoire et comprise entre 200 et 350°C, une pression strictement supérieure à la pression opératoire et comprise entre 0,1 et 3 MPa, une PPH comprise entre 0,1 et 10 $h^{-1}$ et une durée comprise entre 1 et 30 h.

**4.** Procédé selon la revendication 1 dans lequel ledit catalyseur de ladite étape c) est pré-coké avec un alcool primaire substitué en position 2 par un groupement alkyl, à une température moyenne pondérée strictement supérieure à la température opératoire et comprise entre 250 et 450°C, une pression comprise entre 0,1 et 3 MPa, une PPH comprise entre 0,1 et 10 $h^{-1}$ et une durée comprise entre 1 et 30 h, ledit alcool primaire substitué en position 2 par un groupement alkyl comprend moins de 1% masse de composés autre que ledit alcool primaire.

**5.** Procédé selon la revendication 1 dans lequel ledit catalyseur de ladite étape c) est pré-coké avec un alcool primaire substitué en position 2 par un groupement alkyl, à une température moyenne pondérée strictement inférieure à la température opératoire et comprise entre 200 et 350°C, une pression strictement supérieure à la pression opératoire et comprise entre 0,1 et 3 MPa, une PPH comprise entre 0,1 et 10 $h^{-1}$ et une durée comprise entre 1 et 30 h, ledit alcool primaire substitué en position 2 par un groupement alkyl comprend moins de 1% masse de composés autre que ledit alcool primaire.

**6.** Procédé selon la revendication 1 dans lequel ledit catalyseur de ladite étape c) est pré-coké avec l'effluent hydrocarbures lourds issu de l'étape g) de séparation, à une température moyenne pondérée strictement supérieure à la température opératoire et comprise entre 250 et 450°C, une pression comprise entre 0,1 et 3 MPa, une PPH comprise entre 0,1 et 10 $h^{-1}$ et une durée comprise entre 1 et 30 h.

**7.** Procédé selon la revendication 1 dans lequel ledit catalyseur de ladite étape c) est pré-coké avec l'effluent hydrocarbures lourds issu de l'étape g) de séparation, à une température moyenne pondérée strictement inférieure à la température opératoire et comprise entre 200 et 350°C, une pression strictement supérieure à la pression opératoire et comprise entre 0,1 et 3 MPa, une PPH comprise entre 0,1 et 10 $h^{-1}$ et une durée comprise entre 1 et 30 h.

8. Procédé selon l'une des revendications 1 à 7 dans lequel ladite zéolithe comprise dans le catalyseur mis en oeuvre dans ladite étape c) est choisie parmi les zéolithes ferrierite, NU-23, FU-9, ISI-6, ZSM-35 et ZSM-57, prise seule ou en mélange.

9. Procédé selon l'une des revendications 1 à 8 dans lequel un seul four est utilisé pour la chauffe du mélange charge préchauffée/effluent diluant au cours de l'étape b) et pour la surchauffe de la fraction vapeur comprimé, cette dernière étant surchauffée dans la partie chaude du four, tandis que ledit mélange est mis en température dans la partie froide.

**Patentansprüche**

1. Verfahren zur isomerisierenden Dehydratisierung einer Charge, umfassend von 40 bis 100 Gew.-% primären Alkohol, substituiert in Stellung 2 durch eine Alkylgruppe, umfassend mindestens die folgenden Schritte:

a) Setzen der Charge unter Druck, anschließend Erhitzen der komprimierten Charge durch einen Wärmeaustausch mit dem Dehydratisierungsabfluss aus Schritt c) in einem Wärmetauscher, um eine vorerhitzte Charge zu erzeugen;
b) Verdampfen der vorerhitzten Charge durch Mischen mit dem verdünnenden Abfluss aus Schritt f), wobei das Massenflussverhältnis des verdünnenden Abflusses zur vorerhitzten Charge zwischen 5/95 und 60/40 liegt;
c) Dehydratisieren des Abflusses aus Schritt b) in mindestens einem Dehydratisierungsreaktor, der in der Gasphase bei einer gewichteten mittleren Temperatur zwischen 250 und 375°C, bei einem Druck zwischen 0,2 MPa und 1 MPa und bei einem PPH-Wert zwischen 1 und 18 $h^{-1}$ arbeitet, in Anwesenheit eines Katalysators, umfassend einen Zeolithen vom FER-Strukturtyp, wobei der Katalysator zuvor in situ oder ex situ gekokt wird, um einen Dehydratisierungsabfluss zu erzeugen;
d) Abkühlen des Dehydratisierungsabflusses durch mindestens drei aufeinanderfolgende Wärmeaustausche mit mindestens einem Wasserabfluss aus Schritt e), dann der komprimierten Charge aus Schritt a), dann einer Kälteeinrichtung, um einen abgekühlten Abfluss zu erzeugen;
e) Dekantieren des abgekühlten Abflusses in eine wässrige Phase und eine organische Phase, wobei ein Teil der wässrigen Phase abgezogen wird, um außerhalb des Dehydratisierungsverfahrens behandelt zu werden, und der andere Teil, der den Wasserabfluss bildet, über Schritt f) rückgeführt wird;
f) Rückführen des Wasserabflusses aus Schritt e) und mindestens teilweises Verdampfen durch einen Wärmeaustausch in einem Wärmetauscher mit dem Dehydratisierungsabfluss aus Schritt c), Trennen der gegebenenfalls vorliegenden Flüssigfraktion, dann Kompression und Überhitzen der Dampffraktion, um einen verdünnenden Abfluss zu bilden, wobei der verdünnende Abfluss zu Schritt b) rückgeführt wird;
g) Trennen der in Schritt e) extrahierten organischen Phase in mindestens einer Destillationssäule, um einen Abfluss aus Alkenen und einen Abfluss aus schweren Kohlenwasserstoffen zu erzeugen.

2. Verfahren nach Anspruch 1, wobei der Katalysator aus Schritt c) mit einer Charge vorgekokt wird, umfassend primären Alkohol, substituiert in Stellung 2 durch eine Alkylgruppe, bei einem Partialdruck des primären Alkohols streng größer als jener der Charge des Verfahrens nach Anspruch 1, bei einer gewichteten mittleren Temperatur streng größer als die Betriebstemperatur und zwischen 250 und 450°C, einem Druck zwischen 0,1 und 3 MPa, einem PPH-Wert zwischen 0,1 und 10 $h^{-1}$ und einer Dauer zwischen 1 und 30 h.

3. Verfahren nach Anspruch 1, wobei der Katalysator aus Schritt c) mit einer Charge vorgekokt wird, umfassend primären Alkohol, substituiert in Stellung 2 durch eine Alkylgruppe, bei einem Partialdruck des primären Alkohols streng größer als jener der Charge des Verfahrens nach Anspruch 1, bei einer gewichteten mittleren Temperatur streng größer als die Betriebstemperatur und zwischen 200 und 350°C, einem Druck streng größer als der Betriebsdruck und zwischen 0,1 und 3 MPa, einem PPH-Wert zwischen 0,1 und 10 $h^{-1}$ und einer Dauer zwischen 1 und 30 h.

4. Verfahren nach Anspruch 1, wobei der Katalysator aus Schritt c) mit einem primären Alkohol vorgekokt wird, substituiert in Stellung 2 durch eine Alkylgruppe, bei einer gewichteten mittleren Temperatur streng größer als die Betriebstemperatur und zwischen 250 und 450°C, einem Druck zwischen 0,1 und 3 MPa, einem PPH-Wert zwischen 0,1 und 10 $h^{-1}$ und einer Dauer zwischen 1 und 30 h, wobei der primäre Alkohol, substituiert in Stellung 2 durch eine Alkylgruppe, mindestens 1 Masse-% andere Verbindungen als der primäre Alkohol umfasst.

5. Verfahren nach Anspruch 1, wobei der Katalysator aus Schritt c) mit einem primären Alkohol vorgekokt wird, substituiert in Stellung 2 durch eine Alkylgruppe, bei einer gewichteten mittleren Temperatur streng größer als die Betriebstemperatur und zwischen 200 und 350°C, einem Druck streng größer als der Betriebsdruck und zwischen 0,1 und 3 MPa, einem PPH-Wert zwischen 0,1 und 10 h$^{-1}$ und einer Dauer zwischen 1 und 30 h, wobei der primäre Alkohol, substituiert in Stellung 2 durch eine Alkylgruppe, mindestens 1 Masse-% andere Verbindungen als der primäre Alkohol umfasst.

6. Verfahren nach Anspruch 1, wobei der Katalysator aus Schritt c) mit dem Abfluss aus schweren Kohlenwasserstoffen aus dem Trennschritt g) vorgekokt wird, bei einer gewichteten mittleren Temperatur streng größer als die Betriebstemperatur und zwischen 250 und 450°C, einem Druck zwischen 0,1 und 3 MPa, einem PPH-Wert zwischen 0,1 und 10 h$^{-1}$ und einer Dauer zwischen 1 und 30 h.

7. Verfahren nach Anspruch 1, wobei der Katalysator aus Schritt c) mit einem Abfluss aus schweren Kohlenwasserstoffen aus dem Trennschritt g) vorgekokt wird, bei einer gewichteten mittleren Temperatur streng größer als die Betriebstemperatur und zwischen 200 und 350°C, einem Druck streng größer als der Betriebsdruck und zwischen 0,1 und 3 MPa, einem PPH-Wert zwischen 0,1 und 10 h$^{-1}$ und einer Dauer zwischen 1 und 30 h.

8. Verfahren nach einem der Ansprüche 1 bis 7, wobei der Zeolith, der in dem in Schritt c) eingesetzten Katalysator verwendet wird, aus Ferrierit-Zeolithen, NU-23, FU-9, ISI-6, ZSM-35 und ZSM-57, allein oder in Mischung, ausgewählt wird.

9. Verfahren nach einem der Ansprüche 1 bis 8, wobei ein einziger Ofen zum Erhitzen der Mischung der vorerhitzten Charge/des verdünnenden Abflusses während Schritt b) und zum Überhitzen der komprimierten Dampffraktion verwendet wird, wobei diese Letztere im heißen Teil des Ofens überhitzt wird, während die Mischung einer Temperatur im kalten Teil ausgesetzt wird.

## Claims

1. Process for dehydrating and isomerizing a feedstock comprising from 40 to 100% by weight of primary alcohol substituted in position 2 by an alkyl group comprising at least the following steps:

   a) Pressurizing said feedstock then preheating the compressed feedstock by heat exchange with the dehydration effluent obtained from step c) in a heat exchanger to produce a preheated feedstock;
   b) Vaporizing said preheated feedstock by mixing with the diluent effluent obtained from step f), the ratio of the diluent effluent mass flow rate to the preheated feedstock mass flow rate being comprised between 5/95 and 60/40;
   c) Dehydrating the effluent obtained from step b) in at least one dehydration reactor operating in gas phase at a weighted average temperature comprised between 250 and 375°C, at a pressure comprised between 0.2 MPa and 1 MPa and at a WHSV comprised between 1 and 18 h$^{-1}$, in the presence of a catalyst comprising a zeolite of FER structural type, said catalyst being coked beforehand *in-situ* or *ex-situ*, to produce a dehydration effluent;
   d) Cooling said dehydration effluent by at least three successive heat exchanges with at least the water effluent obtained from step e), then said compressed feedstock from step a), then a cold utility to produce a cooled effluent;
   e) Settling of said cooled effluent into an aqueous phase and an organic phase, a part of said aqueous phase being purged to be treated outside of said dehydration process, and the other part forming the water effluent being recycled via step f);
   f) Recycling the water effluent obtained from step e) and at least partial vaporization by heat exchange in a heat exchanger with the dehydration effluent obtained from step c), separation of any liquid fraction that may be present, then compression and superheating of the vapour fraction to form a diluent effluent, said diluent effluent being recycled to step b);
   g) Separation of the organic phase extracted from step e) in at least one distillation column to produce an alkenes effluent and a heavy hydrocarbons effluent.

2. Process according to claim 1, wherein said catalyst from said step c) is pre-coked with a feedstock comprising the primary alcohol substituted in position 2 by an alkyl group at a partial pressure in said primary alcohol strictly greater than that of the feedstock of the process according to claim 1, at a weighted average temperature strictly greater than the operating temperature and comprised between 250 et 450°C, a pressure comprised between 0.1 and 3 MPa, a WHSV comprised between 0.1 and 10 h$^{-1}$ and a duration comprised between 1 and 30 h.

3. Process according to claim 1, wherein said catalyst from said step c) is pre-coked with a feedstock comprising the primary alcohol substituted in position 2 by an alkyl group having a partial pressure in said primary alcohol strictly greater than that of the feedstock of said process according to claim 1, at a weighted average temperature strictly less than the operating temperature and comprised between 200 and 350°C, a pressure strictly greater than the operating pressure and comprised between 0.1 and 3 MPa, a WHSV comprised between 0.1 and 10 h$^{-1}$ and a duration comprised between 1 and 30 h.

4. Process according to claim 1, wherein said catalyst from step c) is pre-coked with a primary alcohol substituted in position 2 by a alkyl group at a weighted average temperature strictly greater than the operating temperature and comprised between 250 and 450°C, a pressure comprised between 0.1 and 3 MPa, a WHSV comprised between 0.1 and 10 h$^{-1}$ and a duration comprised between 1 and 30 h, wherein said primary alcohol substituted in position 2 by an alkyl group comprises less than 1% by weight of compounds other than said primary alcohol.

5. Process according to claim 1, wherein said catalyst from step c) is pre-coked with a primary alcohol substituted in position 2 by a alkyl group, at a weighted average temperature strictly less than the operating temperature and comprised between 200 et 350°C, a pressure strictly greater than the operating pressure and comprised between 0.1 and 3 MPa, a WHSV comprised between 0.1 and 10 h$^{-1}$ and a duration comprised between 1 and 30h, wherein said primary alcohol substituted in position 2 by an alkyl group comprises less than 1% by weight of compounds other than said primary alcohol.

6. Process according to claim 1, wherein said catalyst from step c) is pre-coked with the heavy hydrocarbons effluent obtained from separation step g), at a weighted average temperature strictly greater than the operating temperature and comprised between 250 and 450°C, a pressure comprised between 0.1 and 3 MPa, a WHSV comprised between 0.1 and 10 h$^{-1}$ and a duration comprised between 1 and 30 h.

7. Process according to claim 1, wherein said catalyst from step c) is pre-coked with the heavy hydrocarbons effluent obtained from separation step g), at a weighted average temperature strictly less than the operating temperature and comprised between 200 and 350°C, a pressure strictly greater than the operating pressure and comprised between 0.1 and 3 MPa, a WHSV comprised between 0.1 and 10 h$^{-1}$ and a duration comprised between 1 and 30 h.

8. Process according to one of claims 1 to 7, wherein said zeolite comprised in the catalyst utilized in said step c) is selected from ferrierite, NU-23, FU-9, ISI-6, ZSM-35 and ZSM-57 zeolites, used alone or in a mixture.

9. Process according to one of claims 1 to 8, wherein a single oven is used for heating the preheated feedstock/diluent effluent mixture during step b) and for the superheating of the compressed vapour fraction, the latter being superheated in the hot part of the oven, while said mixture is brought to temperature in the cold part.

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2009079213 A
- EP 2348005 A
- WO 2011089235 A
- WO 2011113834 A
- FR 2733701

**Littérature non-brevet citée dans la description**

- **KOTSARENKO et al.** *Kin. Katal.,* 1983, vol. 24, 877
- **CAÑIZARES et al.** *Applied Catalysis A : General,* 2000, vol. 190, 93-105